(19) Europäisches Patentamt
European Patent Office
Office européen des brevets

(11) **EP 3 731 758 B1**

(12) **FASCICULE DE BREVET EUROPEEN**

(45) Date de publication et mention
de la délivrance du brevet:
**07.01.2026 Bulletin 2026/02**

(21) Numéro de dépôt: **18833076.5**

(22) Date de dépôt: **28.12.2018**

(51) Classification Internationale des Brevets (IPC):
**A61B 6/00** *(2024.01)* **A61B 6/58** *(2024.01)*

(52) Classification Coopérative des Brevets (CPC):
**A61B 6/4441; A61B 6/583;** A61B 6/5205

(86) Numéro de dépôt international:
**PCT/EP2018/097126**

(87) Numéro de publication internationale:
**WO 2019/129879 (04.07.2019 Gazette 2019/27)**

(54) **PROCÉDÉ ET SYSTÈME POUR CALIBRER UN SYSTÈME D'IMAGERIE A RAYONS X**

VERFAHREN UND SYSTEM ZUR KALIBRIERUNG EINES RÖNTGENBILDGEBUNGSSYSTEMS

METHOD AND SYSTEM FOR CALIBRATING AN X-RAY IMAGING SYSTEM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.12.2017 FR 1701389**

(43) Date de publication de la demande:
**04.11.2020 Bulletin 2020/45**

(73) Titulaire: **THALES**
**92190 Meudon (FR)**

(72) Inventeurs:
• **GORGES, Sébastien**
**38430 SAINT JEAN DE MOIRANS (FR)**
• **BERNARD, Guillaume**
**38430 MOIRANS (FR)**
• **GRONDIN, Yannick**
**38240 MEYLAN (FR)**

(74) Mandataire: **Atout PI Laplace**
**Immeuble Up On**
**25 Boulevard Romain Rolland**
**CS 40072**
**75685 Paris Cedex 14 (FR)**

(56) Documents cités:
**EP-A1- 2 868 277** **US-A1- 2002 044 631**
**US-A1- 2017 238 897**

• **MADAN HENNADII ET AL: "Device and methods for gold standard registration of clinical 3D and 2D cerebral angiograms", PROGRESS IN BIOMEDICAL OPTICS AND IMAGING, SPIE - INTERNATIONAL SOCIETY FOR OPTICAL ENGINEERING, BELLINGHAM, WA, US, vol. 9415, 18 March 2015 (2015-03-18), pages 94151G - 94151G, XP060051334, ISSN: 1605-7422, ISBN: 978-1-5106-0027-0, DOI: 10.1117/12.2081908**

**Description**

L'invention concerne un procédé et un système permettant de déterminer, en cours de fonctionnement, les caractéristiques géométriques d'un système de reconstruction d'une image en trois dimensions (3D) à partir d'images acquises avec un système d'imagerie à rayons X. L'invention permet ainsi une calibration en ligne d'un système d'imagerie à rayons X.

Les caractéristiques géométriques sont estimées lors de l'acquisition rotationnelle d'un dispositif de type C-arm.

Il est courant d'utiliser un système de radiologie mobile pour effectuer des procédures chirurgicales ou interventionnelles. Ces systèmes aussi appelés mobile C-arm (ou amplificateur de bloque) permettent au chirurgien d'acquérir des images à rayons X durant l'intervention et de contrôler en temps réel le positionnement des outils utilisés par le chirurgien (cathéter, aiguille, prothèse, ...) de manière minimalement invasive. La plupart de ces systèmes permettent d'obtenir des images en deux dimensions avec un flux d'images vidéo à trente images par seconde. Le praticien utilise ensuite ces images pour effectuer une reconstruction mentale de la morphologie du patient afin de positionner l'outil de manière précise au niveau de la zone à opérer, en temps réel. Plus récemment, des systèmes plus sophistiqués ont fait leur apparition et permettent d'acquérir une image 3D de l'outil utilisé par un chirurgien lors d'une intervention. Le système de radiologie effectue une rotation autour du patient dans le but d'obtenir un ensemble d'images en deux dimensions 2D. Ces images 2D sont ensuite traitées par un algorithme de reconstruction permettant l'obtention d'une image volumique en 3D. Pour reconstruire l'image, l'algorithme a besoin de connaître, pour chaque image 2D, la géométrie exacte du C-arm, à savoir la position du détecteur et de la source de rayons X, par rapport au patient. Les systèmes actuellement utilisés proposent d'effectuer une calibration hors ligne du C-arm durant les phases de maintenance préventive du dispositif, tous les six mois ou un an.

Le brevet US6510241 décrit un procédé pour étalonner un appareil de radiologie, dans lequel un volume virtuel entourant l'objet à imager est généré et décomposé en voxels (pixels en 3D). Le procédé comprend une étape d'acquisition de l'ensemble d'images bidimensionnelles projetées numérotées et une reconstruction de l'image tridimensionnelle à partir de l'image projetée est réalisée.

Le brevet US6320928 décrit une méthode de reconstruction d'images dans laquelle plusieurs images numériques bidimensionnelles d'un objet sont acquises pour différentes positions d'une caméra tournant autour de l'objet. Les images projetées sont calibrées dans un volume contenant l'objet et divisées en voxels dont les coordonnées spatiales sont identifiées dans un cadre de référence d'étalonnage choisi.

Le brevet US6049582 concerne un procédé d'étalonnage de dispositif C-arm pour une reconstruction 3D dans un système d'imagerie comprenant une source d'imagerie et un plan d'imagerie qui utilise une transformation plane pour relier des voxels dans un espace de voxels et des pixels dans le plan d'imagerie.

La demande de brevet EP3141187 concerne une mire de calibration pour calibrer géométriquement un dispositif d'imagerie par rayons X destiné à générer des images tridimensionnelles d'un objet par reconstruction à partir de projections bidimensionnelles dudit objet. La mire de calibration comprend un support volumique muni de marqueurs à absorption radiologique contrastée par rapport au support volumique, les marqueurs étant répartis selon une figure tridimensionnelle. Les marqueurs sont répartis en sous-ensembles de marqueurs distribués selon des droites respectives sensiblement parallèles de façon que des séquences de birapports puissent être construites à partir des sous-ensembles de marqueurs respectifs. Chaque séquence de birapport comprend un unique birapport pour chaque quadruplet de marqueurs dans lequel les marqueurs sont ordonnés selon un ordre dépendant des numéros d'ordre des marqueurs respectifs le long de la droite selon laquelle ils sont alignés, selon un premier sens prédéfini, ledit ordre étant commun à tous les birapports, et lorsqu'un sous-ensemble de marqueurs comprend au moins cinq marqueurs, l'ordre des birapports dans les séquences de birapports respectives suit une règle prédéfinie commune à toutes les séquences de birapports.

Ces systèmes supposent que l'acquisition rotationnelle est suffisamment reproductible pour que la géométrie du C-arm déterminée « hors ligne » soit applicable sur les images acquises durant une intervention chirurgicale.

La mécanique des systèmes a été améliorée afin de rendre l'arceau stable pendant l'acquisition rotationnelle des images 2D. Toutefois, ces améliorations (réduction de jeux mécaniques, utilisation de pièces plus rigides, ...) conduisent à des dispositifs plus onéreux. De plus, il n'est pas toujours trivial d'apporter ces modifications à des dispositifs existants.

D'autres méthodes de l'art antérieur proposent une calibration en ligne.

Une première méthode est basée sur l'utilisation de marqueurs. Une mire de calibration est positionnée sur ou à côté d'un patient pendant l'intervention. Ceci permet d'estimer avec précision et en ligne la géométrie du dispositif sans se soucier du caractère reproductible des conditions de mesure. La demande de brevet US 201000284601 décrit une telle méthode.

Le document de Madan Hennadii et al, intitulé « Device and methods for gold standard regsitration of clinical 3D and 2D cerebral angiograms », progress in biomedical optics and imaging, SPIE, XP 060051334, divulgue une méthode de calibration dans le domaine de l'agiographie pour la construction d'un « gold standard ». L'étape de calibration utilise un groupe de marqueurs disposés dans un bandeau positionné au niveau de la tête du patient.

Toutefois, les méthodes connues de l'art antérieur qui utilisent une mire ne sont pas optimales dans le cadre d'une utilisation chirurgicale ou d'autres applications présentant des contraintes d'utilisation équivalentes, pour les raisons exposées ci-après:

- La mire doit être fabriquée avec précision afin que la position 3D des points soit connue avec précision, ce qui représente un coût,
- La mire elle-même peut être volumineuse et difficile à utiliser lorsque le patient est présent,
- La mire doit avoir été stérilisée, car elle est utilisée dans un environnement stérile, subir un traitement chimique/-thermique avant et après son utilisation.

Un deuxième type de méthode de reconstruction d'images est basé sur l'utilisation d'une image. Ces méthodes exploitent le contenu anatomique d'une image pour effectuer à la fois une reconstruction d'images 3D et une calibration géométrique.

Dans le brevet EP2868277, la méthode utilise des marqueurs, cependant il est nécessaire que les positions 3D des marqueurs soient connues de manière précise pour déterminer les paramètres géométriques.

Résumé de l'invention

L'invention repose sur une nouvelle approche utilisant des marqueurs radio-opaques autocollants sans avoir besoin de connaître la position 3D des marqueurs.

L'invention concerne un procédé pour calculer en cours de fonctionnement les paramètres géométriques d'un système d'imagerie à rayons X selon la revendication 1 ainsi qu'un dispositif correspondant selon la revendication 11.

Le procédé peut comporter une étape de calibration hors ligne afin de calculer initialement les matrices de calibration utilisées lors de l'étape finale de détermination des paramètres de géométrie.

Dans une autre variante, les matrices de projection initiales sont calculées en exploitant les capteurs d'orientation ou les capteurs de positionnement du système.

Les marqueurs peuvent être insérés ou contenus dans des patchs positionnés sur ou à proximité du patient ou de l'objet et les patchs utilisés sont, par exemple, des patchs adhésifs définis comme suit :

- Une bande collante qui sera apposée sur un objet ou un patient,
- Un ensemble de marqueurs radio-opaques répartis sur la surface du patch,
- Une surface extérieure résistante à un fluide.

Il est possible de répartir les marqueurs au niveau d'un patch afin de couvrir l'ensemble de la surface du patch.

Une autre possibilité consiste à utiliser des marqueurs de petite taille répartis sur l'ensemble d'un vêtement compressif avant de couvrir la zone à reconstruire.

Une autre variante consiste à utiliser un ou plusieurs marqueurs anatomiques ou encore à utiliser des marqueurs radio-opaques implantés dans l'anatomie du patient, par exemple dans l'os.

Le procédé peut comporter une étape exploitant les caractéristiques géométriques du système pour reconstruire une image en 3D.

D'autres caractéristiques et avantages de la présente invention apparaîtront mieux à la lecture de la description d'exemples de réalisation donnés à titre illustratif et nullement limitatif, annexée des figures qui représentent :

- Figure 1, un schéma représentant un patient en position,
- Figure 2, un exemple de patch disposé sur le patient, et
- Figure 3, une succession des étapes mises en œuvre par le procédé selon l'invention.

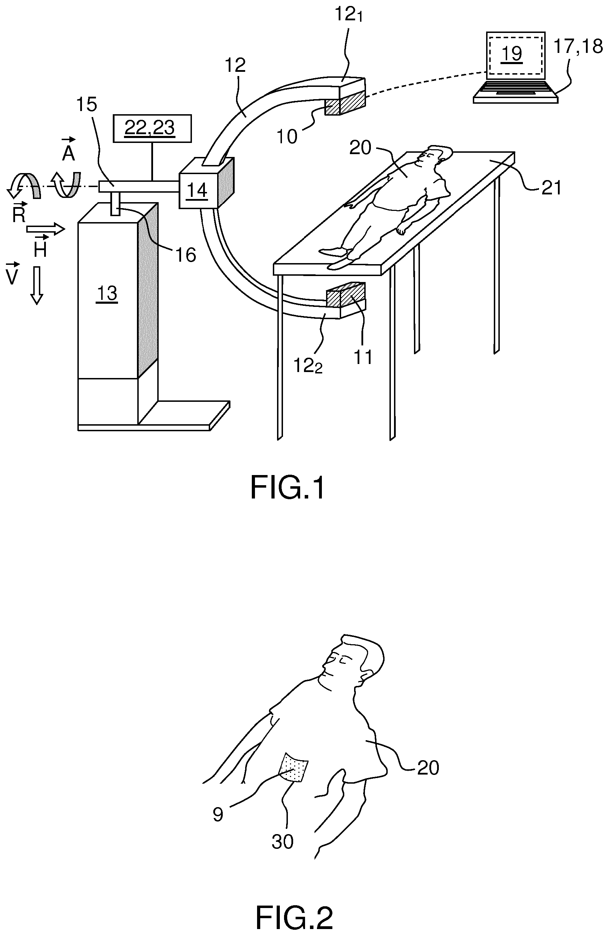

La figure 1 illustre un exemple de dispositif permettant à un praticien de suivre en temps réel la position d'un outil qu'il utilise lors d'une intervention chirurgicale. Le dispositif comporte un système d'imagerie comprenant une source de rayons X, 10, et un détecteur de rayons X, 11. Le dispositif est constitué d'un arceau 12 ou C-arm supportant à une première extrémité $12_1$ la source de rayons X et à une deuxième extrémité $12_2$ le détecteur de rayons X. L'arceau ou bras en arceau 12 est maintenu sur un châssis 13 par une pièce de maintien 14.

Un guide horizontal 15 fixé au châssis 13 via une pièce verticale 16 et à la pièce de maintien 14 de l'arceau, permet d'assurer un mouvement de translation horizontale de l'arceau, flèche $\vec{H}$.

La pièce de maintien 14 permet au bras en arceau 12 d'effectuer un mouvement de type « orbital rotation », selon la flèche $\vec{R}$.

La rotation de la pièce 14 et de l'arceau se traduit par une rotation angulaire selon la flèche $\vec{A}$.

Le mouvement vertical est assuré par la translation horizontale du guide et de la pièce verticale.

Les joints utilisés entre les différents éléments du système permettant les mouvements de rotation précités sont connus de l'homme du métier et ne seront pas détaillés. De même, les mouvements précités du système de radiologie sont connus de l'homme du métier.

Le dispositif comprend également un dispositif de traitement 17 comportant un processeur 18 adapté à exécuter les étapes du procédé selon l'invention, afin de déterminer les caractéristiques géométriques du dispositif lors de l'intervention du chirurgien. Le dispositif peut comporter un écran 19 sur lequel le chirurgien peut visualiser la position de l'outil en temps réel.

Le dispositif est aussi équipé de capteurs d'orientation 22 ou de positionnement 23.

Un patient 20 est positionné sur une table d'opération 21. La figure 2 schématise un exemple de mise en œuvre du procédé où les marqueurs sont incorporés ou intégrés dans un patch.

Le patch 30 est positionné dans la partie supérieure du corps et comprend au moins un marqueur q. Les coordonnées du patch ou la position des patchs ne sont pas connues initialement. Il est possible d'utiliser un ou plusieurs patchs pour la mise en œuvre du procédé selon l'invention.

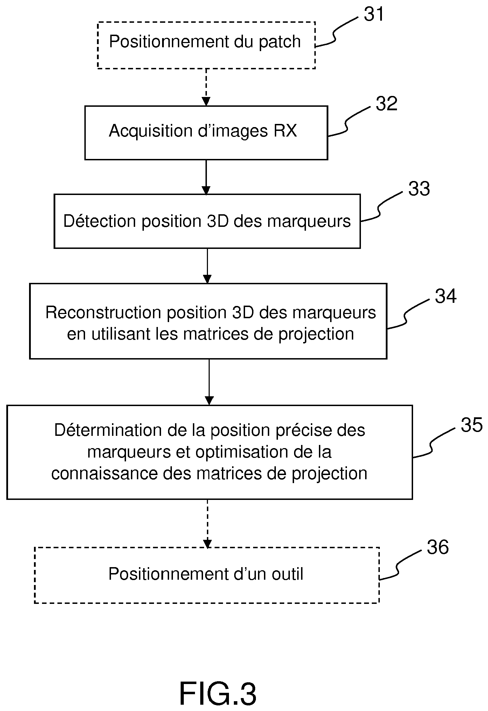

La figure 3 illustre un exemple de succession d'étapes mises en œuvre par le procédé selon l'invention, dans le cas où les marqueurs sont intégrés dans un patch.

Etape 1

Un ou plusieurs patchs 30 intégrant des marqueurs radio-opaques sont apposés sur la peau du patient ou de l'objet 20, au voisinage par exemple d'une zone à opérer, 31. Ceci permettra au praticien d'obtenir avec précision une reconstruction de l'organe qu'il doit opérer.

Etape 2

Plusieurs images 2D sont acquises pour différents points de vue afin d'effectuer une reconstruction 3D, 32. La source de rayons X et le détecteur sont déplacés autour du corps 20 à imager de façon à réaliser plusieurs projections du corps sous des angles de vue différents. Les projections ainsi réalisées seront utilisées pour reconstruire une image tridimensionnelle du corps imagé.

Etape 3

Les marqueurs radio-opaques contenus dans le patch 30 sont détectés dans chaque image 2D acquises au niveau du détecteur de rayons X et appairés, d'une image à l'autre, 33. Des critères de similarités géométriques ou radiométriques sont utilisés pour effectuer l'appariement des marqueurs.

Etape 4

Une reconstruction 3D des marqueurs est effectuée en utilisant une première estimation des matrices de projection, 34. Ces matrices de projection Mi peuvent être déterminées lors d'une calibration préalable hors ligne ou prédites à partir des capteurs de position du système. Ces matrices de projection 4*3 permettent de mettre en correspondance chaque point de l'objet ou du patient dans l'espace 3D, par exemple par rapport au référentiel terrestre, avec sa projection sur un détecteur plan 2D lié au détecteur.

A la fin de cette étape d'appariement, une première estimation de la position 3D des marqueurs est obtenue.

Etape 5

La position 3D des marqueurs et la connaissance des matrices de projection sont alors affinées de manière itérative, 35. Les paramètres géométriques, à savoir les matrices de projection ainsi que la position 3D des marqueurs, sont estimés conjointement en minimisant le critère présenté ci-dessous.

Soit un ensemble de N projections et donc de N matrices à déterminer. Soit un ensemble de L points à reconstruire, le critère est donné par :

$$X, M = argmin \sum_{i=1}^{N} \sum_{j=1}^{L} ||M_i X_j - q_{ij}||$$

où

$q_{ij}$ désigne les coordonnées 2D d'un marqueur de numéro j détecté dans une image i obtenue par le système,
X est l'ensemble des L points 3D à reconstruire, $X_j$ le point de numéro j,
M est l'ensemble des N matrices de projection, $M_i$ la matrice de projection de l'image i.

A la fin de cette étape 35, il est possible de reconstruire, de manière précise, l'image 3D correspondante.

Les images 3D ainsi obtenues pourront être utilisées pour permettre à un praticien de positionner avec précision ses outils en cours d'opération, 36.

Le principe général des méthodes d'ajustement de faisceau est décrit dans le document intitulé « Bundle adjustment - a modern synthesis » de B.trigs, PF Mc Lauchlan, RI Hartley, International Workshop on Vision Algorithms, Corfu, Greece, September 21-22, 1999 Proceedings.

Tout autre algorithme, prenant en entrée les coordonnées d'un marqueur, déterminées par l'exécution du procédé pour en déduire les paramètres géométriques du dispositif, pourra être utilisé.

Selon une variante de réalisation, le procédé comporte une étape préalable de calibration hors ligne conduisant à une géométrie imprécise du C-arm. Les matrices de calibration issues de la calibration « hors ligne » sont utilisées lors de la quatrième étape pour effectuer la première reconstruction.

Les marqueurs utilisés au niveau du patch sont, par exemple, des marqueurs sphériques afin de faciliter la détection. Ils peuvent aussi avoir des formes présentant une symétrie de révolution autour d'axes de symétrie de révolution.

Le ou les patchs contenant les marqueurs peuvent être collés directement sur le patient ou être positionnés à proximité de la zone à imager.

Dans le cas de patchs adhésifs, il est possible d'utiliser un patch défini comme suit :

- Une bande collante qui sera apposée sur la peau d'un patient ou d'un objet, avant l'acquisition des images 2D utiles à la reconstruction des images 3D,
- Un ensemble de marqueurs radio-opaques répartis sur la surface du patch,
- Une surface extérieure résistante à l'eau, le sang et les frottements protégeant le patch de son environnement. La surface extérieure est, par exemple, en matière plastique.

Les patchs autocolllants peuvent être à usage unique.

Les marqueurs sont par exemple répartis de manière à couvrir l'ensemble de la surface du patch.

L'ensemble patch et marqueurs présente, par exemple, une épaisseur d'environ 1mm et une taille approximative de $4 \times 14$ cm.

Les marqueurs peuvent être intégrés dans un tissu étirable, ou « medical strech suit ». Les marqueurs de petites tailles, par exemple des billes opaques, sont par exemple répartis sur l'ensemble du vêtement compressif avant de couvrir l'ensemble à reconstruire, une partie du patient, par exemple. Il est alors possible de faire en même temps, d'une part la calibration du dispositif en utilisant les informations obtenues au cours de la quatrième étape et d'autre part la reconstruction de l'enveloppe (surface 3D) de l'objet à reconstruire. Cette enveloppe servira d'a priori pour la reconstruction 3D.

Selon une variante de réalisation, le procédé utilisera un ou plusieurs marqueurs anatomiques (partie du corps humain caractéristique et radio-opaque) qui correspondent à des points d'intérêts présents dans une image. Les marqueurs seront extraits des images en utilisant un traitement d'images connu de l'homme du métier. Dans cette variante de réalisation, le procédé n'effectuera pas l'étape 31 de positionnement de patch. La première étape consistera à acquérir des images RX.

Dans certains cas, il sera possible d'associer des marqueurs contenus dans des patchs et des marqueurs anatomiques, ces derniers pouvant être implantés dans le corps du patient, par exemple dans un os.

Le procédé selon l'invention permet aussi de calibrer un dispositif C-arm en utilisant un ou plusieurs marqueurs, la position de ces marqueurs n'étant pas initialement connue.

## Revendications

1. - Procédé pour calculer en cours de fonctionnement les paramètres géométriques d'un système d'imagerie à rayons X, un objet ou un patient (20) à observer étant disposé entre la source de rayons X et un détecteur de rayons X ayant traversé l'objet ou le patient, l'objet comprenant une pluralité de marqueurs radio-opaques, le procédé comprenant au moins les étapes suivantes, pour chaque marqueur $X_j$ de l'objet ou du patient :

   • Utiliser le marqueur $X_j$ de position 3D non connue initialement,
   • Acquérir plusieurs images 2D pour plusieurs points de vue du système d'imagerie, (32),

• Détecter la position $q_{ij}$ dudit marqueur $X_j$ dans chacune des images 2D acquises $i$, (33), des critères de similarités géométriques ou radiométriques étant utilisés pour effectuer l'appariement de chaque marqueur $X_j$ d'une image à l'autre, et

• Estimer les matrices de projection $M_i$ des images $i$ correspondant aux projections de l'objet sous des angles de vues différents et reconstruire en 3D la position dudit marqueur à partir de l'estimation des matrices de projection (34),

le procédé comprenant en outre une étape (35) d'estimation itérative conjointe de $X$ l'ensemble des positions 3D des marqueurs et de $M$ l'ensemble des $N$ matrices de projection, en minimisant un critère, le critère étant :

$$X, M = argmin \sum_{i=1}^{N} \sum_{i=1}^{L} ||M_i X_j - q_{ij}||$$

le procédé comprenant une reconstruction en 3D de l'image de l'objet ou du patient, en réponse à la fin de ladite étape (35) d'estimation itérative.

2. - Procédé selon la revendication 1 **caractérisé en ce qu'**il comporte une étape de calibration hors ligne afin de calculer les matrices de projection initiales.

3. - Procédé selon la revendication 1 **caractérisé en ce que** les matrices de projection initiales sont calculées en exploitant les capteurs d'orientation ou de positionnement du système.

4. - Procédé selon l'une des revendications 1 à 3 **caractérisé en ce que** les marqueurs sont contenus dans un patch adhésif positionné sur ou à proximité du patient ou de l'objet, défini comme suit :

   • Une bande collante,
   • Un ensemble de marqueurs radio-opaques répartis sur la surface du patch,
   • Une surface extérieure résistante à un fluide.

5. - Procédé selon l'une des revendications 1 à 3 **caractérisé en ce que** l'on répartit les marqueurs au niveau d'un patch afin de couvrir l'ensemble de la surface du patch.

6. - Procédé selon l'une des revendications 1 à 4 **caractérisé en ce que** l'on utilise des marqueurs intégrés dans un tissu étirable.

7. - Procédé selon l'une des revendications 1 à 5 **caractérisé en ce que** l'on utilise des marqueurs de petite taille répartis sur l'ensemble d'un vêtement compressif avant de couvrir une partie d'un patient à reconstruire.

8. - Procédé selon l'une des revendications 1 à 3 **caractérisé en ce que** l'on utilise au moins un marqueur anatomique.

9. - Procédé selon l'une des revendications 1 à 3 **caractérisé en ce que** l'on utilise au moins un marqueur radio-opaque implanté dans l'anatomie du patient, tel que dans l'os.

10. - Procédé selon l'une des revendications 1 à 9 **caractérisé en ce qu'**il comporte une étape exploitant les caractéristiques géométriques du système pour reconstruire une image en 3D.

11. - Dispositif pour calculer en cours de fonctionnement les paramètres géométriques d'un système d'imagerie à rayons X, un objet ou un patient (20) à observer étant disposé entre la source de rayons X et un détecteur de rayons X ayant traversé l'objet ou le patient, le dispositif comprenant en outre au moins un dispositif de traitement (17) comprenant un processeur (18) adapté à exécuter les étapes du procédé selon l'une des revendications 1 à 10.

**Patentansprüche**

1. Verfahren zum Berechnen der geometrischen Parameter eines Röntgenbildgebungssystems X während des Betriebs, wobei ein zu beobachtendes Objekt oder ein Patient (20) zwischen der Röntgenquelle X und einem Röntgendetektor X angeordnet ist, der das Objekt oder den Patienten durchquert hat, wobei das Objekt eine Vielzahl von strahlenundurchlässigen Markern umfasst, wobei das Verfahren für jeden Marker $X_j$ des Objekts oder Patienten mindestens die folgenden Schritte umfasst:

- Verwenden des Markers $X_j$ für die anfänglich unbekannte 3D-Position,
- Erfassen mehrerer 2D-Bilder für mehrere Blickwinkel des Bildgebungssystems (32),
- Erkennen der Position $q_{ij}$ des Markers $X_j$ in jedem der erfassten 2D-Bilder i, (33), wobei geometrische oder radiometrische Ähnlichkeitskriterien verwendet werden, um die Zuordnung jedes Markers $X_j$ von einem Bild zum anderen durchzuführen, und
- Schätzen der Projektionsmatrizen $M_i$ der Bilder i, die den Projektionen des Objekts unter verschiedenen Blickwinkeln entsprechen, und Rekonstruieren der Position des Markers in 3D anhand der Schätzung der Projektionsmatrizen (34),

wobei das Verfahren weiter einen Schritt (35) der iterativen gemeinsamen Schätzung von X der Gesamtheit der 3D-Positionen der Marker und von M der Gesamtheit der N Projektionsmatrizen umfasst, wobei ein Kriterium minimiert wird, wobei das Kriterium lautet:

$$X, M = argmin \sum_{i=1}^{N} \sum_{i=1}^{L} \|M_i X_j - q_{ij}\|$$

wobei das Verfahren eine 3D-Rekonstruktion des Bildes des Objekts oder des Patienten als Reaktion auf das Ende des Schritts (35) der iterativen Schätzung umfasst.

2. Verfahren nach Anspruch 1, **dadurch gekennzeichnet, dass** es einen Offline-Kalibrierungsschritt umfasst, um die anfänglichen Projektionsmatrizes zu berechnen.

3. Verfahren nach Anspruch 1, **dadurch gekennzeichnet, dass** die anfänglichen Projektionsmatrizen berechnet werden, indem die Ausrichtungs- oder Positionierungssensoren des Systems genutzt werden.

4. Verfahren nach einem der Ansprüche 1 bis 3, **dadurch gekennzeichnet, dass** die Marker in einem Klebepflaster enthalten sind, das auf oder in der Nähe des Patienten oder Objekts positioniert ist, definiert wie folgt:

- ein Klebeband,
- ein Satz von strahlenundurchlässigen Markern, die über die Oberfläche des Pflasters verteilt sind.
- eine äußere Oberfläche, die gegen ein Fluid beständig ist.

5. Verfahren nach einem der Ansprüche 1 bis 3, **dadurch gekennzeichnet, dass** die Marker im Bereich eines Pflasters verteilt werden, um die gesamte Oberfläche des Pflasters abzudecken.

6. Verfahren nach einem der Ansprüche 1 bis 4, **dadurch gekennzeichnet, dass** Marker verwendet werden, die in ein dehnbares Gewebe integriert sind.

7. Verfahren nach einem der Ansprüche 1 bis 5, **dadurch gekennzeichnet, dass** kleine Marker verwendet werden, die über das gesamte Kompressionskleidungsstück verteilt sind, bevor ein Teil eines Patienten, der rekonstruiert werden soll, bedeckt wird.

8. Verfahren nach einem der Ansprüche 1 bis 3, **dadurch gekennzeichnet, dass** mindestens ein anatomischer Marker verwendet wird.

9. Verfahren nach einem der Ansprüche 1 bis 3, **dadurch gekennzeichnet, dass** mindestens ein strahlenundurchlässiger Marker verwendet wird, der in die Anatomie des Patienten, beispielsweise in den Knochen, implantiert wird.

10. Verfahren nach einem der Ansprüche 1 bis 9, **dadurch gekennzeichnet, dass** es einen Schritt umfasst, der die geometrischen Eigenschaften des Systems nutzt, um ein 3D-Bild zu rekonstruieren.

11. Vorrichtung zum Berechnen der geometrischen Parameter eines Röntgenbildgebungssystems X während des Betriebs, wobei ein zu beobachtendes Objekt oder ein Patient (20) zwischen der Röntgenquelle X und einem Röntgendetektor X angeordnet ist, nachdem die Röntgenstrahlen das Objekt oder den Patienten durchquert haben, wobei die Vorrichtung weiter mindestens eine Verarbeitungsvorrichtung (17) umfasst, die einen Prozessor (18) umfasst, der dazu angepasst ist, die Schritte des Verfahrens nach einem der Ansprüche 1 bis 10 auszuführen.

**Claims**

1. Method for calculating during use the geometric parameters of an x-ray imaging system, an object or a patient (20) to be observed being placed between the x-ray source and a detector of x-rays having passed through the object or patient, the object comprising a plurality of radiopaque markers, the method comprising at least the following steps, for each marker $X_j$ of the object or patient:

   • Using the marker $X_j$ of initially unknown 3D position,
   • Acquiring a plurality of 2D images for a plurality of viewpoints of the imaging system (32),
   • Detecting the position $q_{ij}$ of said marker $X_j$ in each of the acquired 2D images i (33), criteria of geometric or radiometric similarities being used to match each marker $X_j$ of one image to another, and
   • Estimating the projection matrices $M_i$ of the images i corresponding to the projections of the object at various viewing angles and reconstructing in 3D the position of said marker on the basis of the estimation of the projection matrices (34),
   the method further comprising a step (35) of jointly iteratively estimating from $X$, all of the 3D positions of the markers and from $M$, all of the $N$ projection matrices, by minimising a criterion, the criterion being:

$$X, M = argmin \sum_{i=1}^{N} \sum_{i=1}^{L} \|M_i X_j - q_{ij}\|$$

   the method comprising a reconstruction in 3D of the image of the object or patient, in response to the end of said iterative estimation step (35).

2. Method according to claim 1, **characterised in that** it comprises an off-line calibration step, in order to calculate the initial projection matrices.

3. Method according to claim 1, **characterised in that** the initial projection matrices are calculating by utilising the system orientation or positioning sensors.

4. Method according to any one of claims 1 to 3, **characterised in that** the markers are contained in an adhesive patch positioned on or in the proximity of the patient or object, defined as follows:

   • an adhesive strip,
   • a set of radiopaque markers distributed over the surface of the patch,
   • an outer surface resistant to a fluid.

5. Method according to any one of claims 1 to 3, **characterised in that** the markers are distributed at a patch, in order to cover the entire surface of the patch.

6. Method according to any one of claims 1 to 4, **characterised in that** markers integrated in a stretch fabric are used.

7. Method according to any one of claims 1 to 5, **characterised in that** small markers are used, distributed over the entirety of a compressive piece of clothing before covering a part of a patient to be reconstructed.

8. Method according to any one of claims 1 to 3, **characterised in that** at least one anatomic marker is used.

9. Method according to any one of claims 1 to 3, **characterised in that** at least one radiopaque marker is used, implanted in the anatomy of the patient, such as in the bone.

10. Method according to any one of claims 1 to 9, **characterised in that** it comprises a step utilising the geometric features of the system to reconstruct an image in 3D.

11. Device for calculating during use the geometric parameters of an x-ray imaging system, an object or a patient (20) to be observed being placed between the x-ray source and a detector of x-rays having passed through the object or patient, the device further comprising at least one processing device (17) comprising a processor (18) adapted to executing the steps of the method according to any one of claims 1 to 10.

FIG.1

FIG.2

FIG.3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 6510241 B
- US 6320928 B
- US 6049582 A
- EP 3141187 A
- US 201000284601 A
- EP 2868277 A

**Littérature non-brevet citée dans la description**

- **MADAN HENNADII et al.** Device and methods for gold standard regsitration of clinical 3D and 2D cerebral angiograms. *SPIE*
- **DE B.TRIGS** ; **PF MC LAUCHLAN** ; **RI HARTLEY**. Bundle adjustment - a modern synthesis. *International Workshop on Vision Algorithms*, 21 September 1999